**Europäisches Patentamt**

**European Patent Office**

**Office européen des brevets**

(19)

(11) Veröffentlichungsnummer: **0 142 736**
**B1**

(12) **EUROPÄISCHE PATENTSCHRIFT**

(45) Veröffentlichungstag der Patentschrift:
13.05.87

(51) Int. Cl.⁴: **A 61 M 5/14**

(21) Anmeldenummer: **84112824.2**

(22) Anmeldetag: **24.10.84**

(54) **Druckinfusionsapparate mit einer Einmalspritze.**

(30) Priorität: **09.11.83 DE 3340511**

(43) Veröffentlichungstag der Anmeldung:
**29.05.85 Patentblatt 85/22**

(45) Bekanntmachung des Hinweises auf die Patenterteilung:
**13.05.87 Patentblatt 87/20**

(84) Benannte Vertragsstaaten:
**AT BE CH DE FR GB IT LI LU NL SE**

(56) Entgegenhaltungen:
**DE - A - 3 307 810**
**GB - A - 2 025 368**
**GB - A - 2 115 495**
**US - A - 3 884 228**
**US - A - 4 126 132**

(73) Patentinhaber: **INTERMEDICAT GMBH,**
**Gerliswilstrasse 74, CH-6020 Emmenbrücke (CH)**

(72) Erfinder: **Mardorf, Robert, Zur Schlade 29,**
**D-3508 Melsungen (DE)**
Erfinder: **von der Haar, Friedrich, Prof. Dr., Tilsiter**
**Strasse 2, D-3508 Melsungen (DE)**

(74) Vertreter: **von Kreisler, Alek, Dipl.-Chem. et al,**
**Deichmannhaus am Hauptbahnhof, D-5000 Köln 1 (DE)**

ACTORUM AG

**Beschreibung**

Die Erfindung betrifft einen Druckinfusionsapparat mit einer Einmalspritze, die einen Zylinder aufweist, in dem ein von dem Druckinfusionsapparat verschiebbarer Kolben angeordnet ist.

Bekannt sind Druckinfusionsapparate (DE-A-3 150 623) für medizinische Anwendungen, bei denen zur Durchführung von Dauerinfusionen eine Spritze langsam und kontinuierlich ausgedrückt wird. Die Spritze wird mit ihrem Zylinder an einem Halter des Druckinfusionsapparates festgelegt, während ein Schieber die Kolbenstange mit einer definierten Geschwindigkeit in Richtung auf den Halter vorschiebt, so dass die in der Spritze enthaltene Infusionslösung stetig und mit einer vorbestimmten wählbaren Rate aus dem Zylinder ausgeschoben wird. Wenn in der Flüssigkeitsleitung, die vom Spritzenauslass zum Patienten führt, eine Verstopfung auftritt oder wenn der Kolben seine Endstellung erreicht und gegen die auslassseitige Zylinderbrust drückt, muss ein Alarmsignal erzeugt werden, dass dem Klinikpersonal anzeigt, dass die Infusion gestört ist bzw. dass der Zylinder leergeworden ist und ggf. durch einen neuen Zylinder ersetzt werden muss. Die Erzeugung des Alarmsignals erfolgt bei dem bekannten Druckinfusionsgerät dadurch, dass der Halter, an dem der Zylinder befestigt ist, eine Verschiebebewegung ausführen kann und von einer Feder in Richtung auf den Schieber vorgespannt ist. Bei einer Obstruktion der vom Zylinder zum Patienten führenden Flüssigkeitsleitung bzw. wenn der Kolben gegen die Zylinderbrust stösst, wird durch die auf den Zylinder ausgeübte Kraft der Halter entgegen der Wirkung der Feder verschoben, wodurch ein Mikroschalter betätigt wird, der den Alarm auslöst. Dieses Prinzip hat den Nachteil, dass der «Drucksensor» am Druckinfusionsapparat eine aufwendige Konstruktion hat und eine bewegliche Anbringung des Halters erfordert. Hinsichtlich der Ansprechgenauigkeit hat es sich als nachteilig erwiesen, dass der Reibwert zwischen Kolben und Zylinder den Schaltpunkt der Alarmauslösung beeinflusst. Da dieser Reibwert von Spritze zu Spritze unterschiedlich sein kann, ist nicht gewährleistet, dass die Alarmauslösung stets bei demselben Flüssigkeitsdruck im Zylinder erfolgt. Eine ähnliche Abhängigkeit von den Exemplareigenschaften der Spritze würde sich auch dann ergeben, wenn der Überlastfall durch Messung der Stromaufnahme des Antriebsmotors des Druckinfusionsapparates erfolgen würde.

Der Erfindung liegt die Aufgabe zugrunde, eine Einmalspritze der eingangs genannten Art zu schaffen, die es ermöglicht, mit geringem Aufwand einen Überlastfall aufgrund der im Zylinder herrschenden Druck- bzw. Kraftverhältnisse festzustellen.

Zur Lösung dieser Aufgabe ist erfindungsgemäss vorgesehen, dass

a) der Zylinder ein verformbares Wandteil aufweist, dessen Position sich in Abhängigkeit von dem im Zylinder herrschenden Flüssigkeitsdruck und/oder durch einen Druck des Kolbens verändert,

b) ein Stellungssensor am Druckinfusionsapparat angeordnet ist, der durch die Position des verformbaren Wandteils betätigt wird, und

c) der Stellungssensor bei der Druckverformung des verformbaren Wandteils ein Alarmsignal auslöst.

Nach der Erfindung ist an dem Zylinder ein verformbares Wandteil vorgesehen, das durch den Flüssigkeitsdruck bzw. durch direkte Berührung mit dem Kolben verformbar ist und bei Verformung den Stellungssensor betätigt. Dies hat den Vorteil, dass die Spritze durch einen Halter festgelegt werden kann, der seinerseits fest mit dem Gehäuse des Druckinfusionsapparates verbunden ist und nicht verschiebbar angebracht sein muss. Auch der Stellungssensor ist fest mit dem Gehäuse des Druckinfusionsapparates verbunden, wobei natürlich eine Justagemöglichkeit zur Änderung der Position des Stellungssensors vorgesehen sein kann. Durch einen Druck im Inneren des Zylinders wird durch das verformbare Wandteil der Stellungssensor, der sich vorzugsweise am auslassseitigen Ende des Zylinders befindet, betätigt. Andererseits kann das verformbare Wandteil auch durch direkten Berührungskontakt mit dem Kolben bewegt werden, um den Stellungssensor zu betätigen.

Gemäss einer bevorzugten Ausführungsform der Erfindung ist das verformbare Wandteil die auslassseitige Stirnwand (Zylinderbrust) des Zylinders. Die Zylinderbrust ist als verformbares Wandteil besonders geeignet, da sie sich einerseits am vorderen (auslassseitigen) Zylinderende befindet und da andererseits im Falle ihrer Verformung die lichte Weite des Zylinders nicht verändert und die Abdichtung gegenüber dem Kolben nicht verschlechtert wird.

Andererseits kann das verformbare Wandteil auch in der Umfangswand des Zylinders angeordnet sein. In diesem Fall hat das verformbare Wandteil gewissermassen die Funktion einer Blase, die in das Innere des Zylinders gerichtet sein kann und bei einem zu grossen Flüssigkeitsdruck nach aussen verformt wird, um den Stellungssensor zu betätigen. Eine solche Verformung der Blase nach aussen erfolgt auch, wenn der Kolben die Blase erreicht und diese bei seiner Vorwärtsbewegung nach aussen drückt. Hierzu eignet sich auch jede andere flexible Querschnittsverengung des Zylinders. Wenn der Sensor nur auf den Flüssigkeitsdruck, nicht aber auf die Stellung des Kolbens ansprechen soll, kann das flexible Wandteil jede beliebige Form haben, wobei nur sichergestellt sein muss, dass es sich bei einem übermässigen Flüssigkeitsdruck nach aussen aufweitet.

Im einfachsten Fall ist der Stellungssensor ein Schalter, der durch das verformbare Wandteil betätigt wird. Es kann jedoch zweckmässig sein, einen Stellungssensor zu verwenden, der ein der Position des verformbaren Wandteils proportionales Ausgangssignal liefert. Auf diese Weise kann eventuell ein Voralarm gegeben werden oder der Druck bzw. die Position des Kolbens kann angegeben werden, so dass das Klinikpersonal auch schon vor dem akuten Alarmzustand die Grösse des betreffenden Wertes überwachen kann.

Im folgenden wird unter Bezugnahme auf die Zeichnungen ein Ausführungsbeispiel der Erfindung näher erläutert.

Es zeigen:

Fig. 1 eine Seitenansicht eines Druckinfusionsapparates mit daran aufgehängter Spritze kurz vor der Betätigung des Stellungssensors und

Fig. 2 eine Darstellung der Spritze bei Betätigung des Stellungssensors infolge eines zu hohen Flüssigkeitsdruckes.

Der dargestellte Druckinfusionsapparat weist ein langgestrecktes rohrförmiges Gehäuse 10 auf, an dessen Unterseite ein erster Halter 11 fest angebracht ist. In dem Gehäuse 10 ist ein Schieber 12 geführt, der aus dem freien Ende des Gehäuses herausragt und einen nach unten ragenden zweiten Halter 13 trägt. Die Linearbewegung des Schiebers 12 wird von einem am Gehäuse 10 befestigten Motor 14 angetrieben. Der Halter 11 weist eine seitliche Öffnung auf, in die der Zylinder 19 der Spritze 16 seitlich eingeschoben wird. Ein Flansch 17 am rückwärtigen Ende des Zylinders 19 stützt sich an der Rückseite des Halters 11 ab. Der Flansch 17 wird von einem Ansatz 27 des Halters 11 umgriffen, so dass der Zylinder 19 in Richtung seiner Längsachse fixiert ist.

Die Spritze 16 besteht aus einem Zylinder 19 und einem Kolben 20, der in dem Zylinder verschiebbar ist. Am Hals 15 des Zylinders 19 ist ein abstehender Flansch 18 angebracht. Der Kolben 20 weist eine aus dem rückwärtigen Ende des Zylinders 19 herausragende Kolbenstange 21 auf, die an ihrem äusseren Ende so profiliert ist, dass sie an dem Halter 13 festgelegt werden kann.

An einem Anschlussstutzen 22, der dem Hals 15 angeformt ist, wird ein (nicht dargestellter) Schlauch angeschlossen, der von der Spritze 16 zum Patienten führt. Der Kolben 20 befindet sich am Beginn der Infusion in einer Stellung in der Nähe des rückwärtigen Zylinderendes. Der Motor 14 treibt den Schieber 12 derart an, dass der Kolben 20 in dem Zylinder 19 langsam vorgeschoben wird und hierbei den Spritzeninhalt durch den Stutzen 22 ausschiebt.

Die Zylinderbrust, d.h. die auslassseitige Stirnwand des Zylinders 19, ist als verformbares Wandteil 23 ausgebildet. Dieses Wandteil 23 kann beispielsweise eine geringere Wandstärke aufweisen als die übrigen Wandteile des Zylinders. Das verformbare Wandteil 23, das im wesentlichen radial, d.h. nach Art eines Flansches, verläuft, geht an seinem äusseren Rand über einen Wulst 24 in die Umfangswand des Zylinders 19 über und grenzt mit seinem inneren Rand an die relativ dicke Wand des Halses 15 an. Auf diese Weise ist das verformbare Wandteil 23 durch den Wulst 24 und den Hals 15 in definierter Weise nach aussen und innen begrenzt.

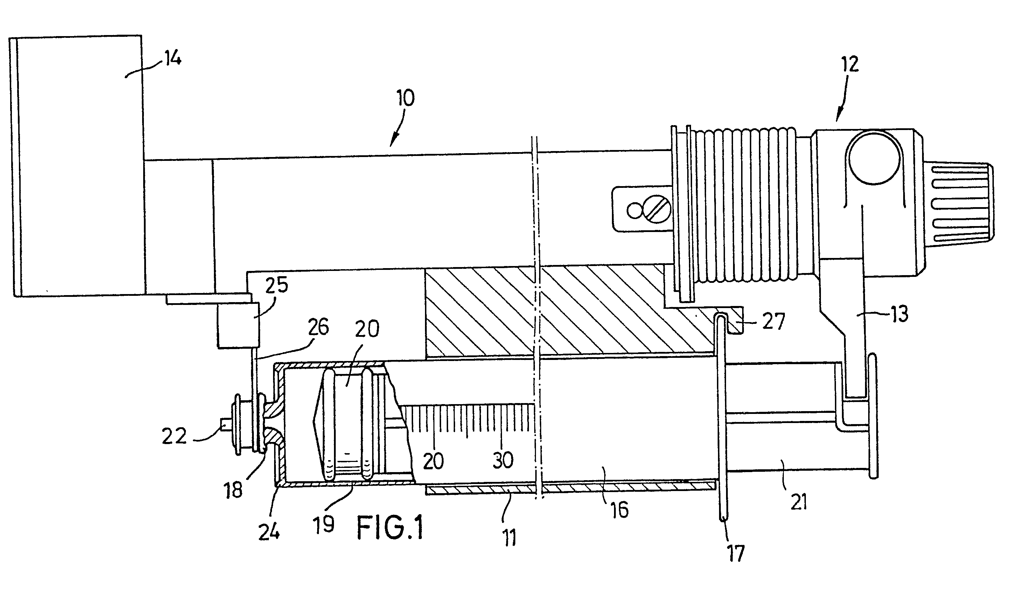

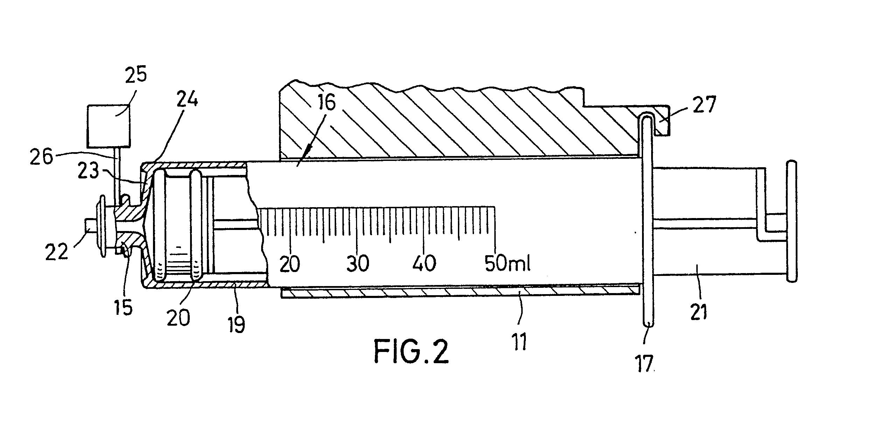

An dem Gehäuse 10 bzw. Halter 11 ist ein Stellungssensor 25 befestigt, der mit einem gabelförmigen Taststift 26 den vor dem Flansch 18 liegenden Bereich des Halses 15 umgreift. Wird das flexible Wandteil 23 in der in Fig. 2 dargestellten Weise verformt, indem entweder im Innern des Zylinders 19 ein zu hoher Flüssigkeitsdruck auftritt oder indem dieses Wandteil 23 durch Berührung mit dem Kolben 20 von diesem vorgeschoben wird, dann wird der Taststift 26 von der flexiblen Wand 23 bzw. dem vorgeschobenen Hals 15 mitgenommen und in Richtung auf das auslassseitige Spritzenende verschoben. Hierdurch kann ein Schaltvorgang ausgeführt werden. Bei dem beschriebenen Ausführungsbeispiel bewegt sich die Wand 23 mit dem Hals 15 in Achsrichtung der Spritze, d.h. der Hals 15 wird durch einen im Zylinder 19 herrschenden Überdruck (in der Zeichnung nach links) vorgeschoben, so wie dies in Fig. 2 dargestellt ist. Die flexible Wand 23, die im Ruhezustand (Fig. 1) die Form einer flachen radialen Scheibe hat, verformt sich kegelstumpfförmig (Fig. 2) und verschiebt den Hals 15 und den Taststift 26. Die Verformung kann entweder durch einen hohen Flüssigkeitsdruck im Zylinder auftreten oder durch mechanisches Ausformen durch die kegelförmige Spritze des Kolbens 20.

Da bei der Druckverformung die Verschiebung der verformbaren Wand 23 proportional zur Grösse des Flüssigkeitsdruckes im Zylinder ist, kann der Stellungssensor 25 derart ausgebildet sein, dass er ein elektrisches Signal erzeugt, dessen Grösse der Stellung des Taststiftes 26 proportional ist. Die gesamte Spritze, die als Einmalspritze ausgebildet ist, besteht aus Kunststoff, wobei die verformbare Wand 23 dünnwandiger ausgebildet ist als die übrigen Wandteile.

**Patentansprüche**

1. Druckinfusionsapparat mit einer Einmalspritze, die einen Zylinder (19) aufweist, in dem ein von dem Druckinfusionsapparat (10) verschiebbarer Kolben (20) angeordnet ist, dadurch gekennzeichnet, dass

a) der Zylinder (19) ein verformbares Wandteil (23) aufweist, dessen Position sich in Abhängigkeit von dem im Zylinder herrschenden Flüssigkeitsdruck und/oder durch einen Druck des Kolbens (20) verändert,

b) ein Stellungssensor (25) am Druckinfusionsapparat angeordnet ist, der durch die Position des verformbaren Wandteils betätigt wird, und

c) der Stellungssensor (25) bei der Druckverformung des verformbaren Wandteils ein Alarmsignal auslöst.

2. Einmalspritze nach Anspruch 1, dadurch gekennzeichnet, dass das verformbare Wandteil (23) die auslassseitige Stirnwand des Zylinders (19) ist.

3. Einmalspritze nach Anspruch 1, dadurch gekennzeichnet, dass das verformbare Wandteil in der Umfangswand des Zylinders angeordnet ist.

4. Einmalspritze nach einen der Ansprüche 1 bis 3, dadurch gekennzeichnet, dass der Stellungssensor (25) ein der Position des verformbaren Wandteils (23) proportionales Ausgangssignal liefert.

5. Einmalspritze nach einen der Ansprüche 1 bis 4, dadurch gekennzeichnet, dass der Zylinder (19) an dem Druckinfusionsapparat (10) mit einem Halter (11) festlegbar ist, der an einem in Ausstossrichtung der Flüssigkeit hinter dem flexiblen Wandteil (23) angeordneten Flanschteil (17) des Zylinders (19) angreift, und dass ein Taststift (26) des Stellungssensors (25) von dem Hals (15) des Zylinders (19) verschiebbar ist.

**Claims**

1. A pressure infusion apparatus with a disposable syringe having a cylinder (19) in which a piston (20) which can be moved by the pressure infusion apparatus (10) is arranged, characterised in that

a) the cylinder (19) has a deformable wall portion (23) whose position changes as a function of the fluid pressure prevailing in the cylinder and/or by means of a pressure by the plunger (20),

b) a position sensor (25) is arranged on the pressure infusion apparatus and is actuated by the position of the deformable wall portion, and

c) the position sensor (25) triggers an alarm signal during pressure deforming of the deformable wall portion.

2. A disposable syringe according to claim 1, characterised in that the deformable wall portion (23) is the outlet end wall of the cylinder (19).

3. A disposable syringe according to claim 1, characterised in that the deformable wall portion is arranged in the peripheral wall of the cylinder.

4. A disposable syringe according to one of claims 1 to 3, characterised in that the position sensor (25) transmits an output signal proportional to the position of the deformable wall portion (23).

5. A disposable syringe according to one of claims 1 to 4, characterised in that the cylinder (19) can be fixed on the pressure infusion apparatus (10) by a holder (11) which acts on a flange number (17) of the cylinder (19) arranged in the fluid discharge direction behind the flexible wall portion (23), and in that a scanning pin (26) of the position sensor (25) can be moved by the neck (15) of the cylinder (19).

**Revendications**

1. Appareil d'injection sous pression comportant une seringue à usage unique, qui possède un cylindre (19), dans lequel se trouve disposé un piston (20) qui peut être déplacé par l'appareil d'injection sous pression (10), caractérisé en ce que:

a) le cylindre (19) comporte un élément de paroi déformable (23), dont la position varie en fonction de la pression de liquide régnant dans le cylindre et/ou sous l'effet d'une pression du piston (20),

b) sur l'appareil d'injection sous pression se trouve disposé un capteur de position (25), qui est actionné par la position de l'élément de paroi déformable, et

c) le capteur de position (25) déclenche un signal d'alarme lors de la déformation sous pression de l'élément de paroi déformable.

2. Seringue à usage unique selon la revendication 1, caractérisée en ce que l'élément de paroi déformable (23) constitue la paroi avant, située côté sortie, du cylindre (19).

3. Seringue à usage unique selon la revendication 1, caractérisée en ce que la paroi déformable est disposée dans la paroi périphérique du cylindre.

4. Seringue à usage unique selon l'une quelconque des revendications 1 à 3, caractérisée en ce que le capteur de position (25) fournit un signal de sortie proportionnel à la position de l'élément de paroi déformable (23).

5. Seringue à usage unique selon l'une quelconque des revendications 1 à 4, caractérisée en ce que le cylindre (19) peut être fixé sur l'appareil d'injection sous pression (10) au moyen d'un support (11) qui est accroché à un élément à bride (17) du cylindre (19), disposé à l'arrière de l'élément de paroi flexible (23) dans la direction de refoulement du liquide, et qu'un palpeur (26) du capteur de position (25) peut être déplacé par le col (15) du cylindre (19).

FIG.1

FIG.2